(19) 

**Europäisches Patentamt**
**European Patent Office**
**Office européen des brevets**

(11) **EP 4 173 572 A1**

(12) # EUROPEAN PATENT APPLICATION

(43) Date of publication:
03.05.2023 Bulletin 2023/18

(21) Application number: 21315228.3

(22) Date of filing: 29.10.2021

(51) International Patent Classification (IPC):
**A61B 8/08** (2006.01)  **G01S 7/52** (2006.01)
**G01S 15/10** (2006.01)  **G01S 15/89** (2006.01)

(52) Cooperative Patent Classification (CPC):
**G01S 15/8959; A61B 8/481; A61B 8/5207;**
**G01S 7/52026; G01S 7/52039; G01S 15/8927**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **SuperSonic Imagine**
**13290 Aix en Provence (FR)**

(72) Inventor: **Lambert, William**
**13008 Marseille (FR)**

(74) Representative: **Paustian & Partner Patentanwälte**
**mbB**
**Oberanger 32**
**80331 München (DE)**

(54) **METHOD AND SYSTEM FOR DETERMINING A PHYSICAL CHARACTERISTIC OF A MEDIUM**

(57)    The invention relates to a method method for determining a physical characteristic of a medium (11), comprising:

processing (d) ultrasound signal data of the medium associated to at least three emitted ultrasound pulses for respectively providing at least three in-phase and quadrature phase (IQ) data sets $I_1(r)$, $I_2(r)$, $I_3(r)$, the at least three emitted ultrasound pulses comprising a first emitted pulse having a first intensity and at least two supplementary emitted pulses having each a second intensity, wherein a sum of the second intensities corresponds to the first intensity, determining (e) the physical characteristic as a function of:

a first phase lag between the first IQ data set $I_1(r)$ and a sum of the at least two further IQ data sets $I_2(r)$, $I_3(r)$, and/or

a second phase lag between the at least two further IQ data sets $I_2(r)$, $I_3(r)$.

Fig. 1

EP 4 173 572 A1

**Description**

FIELD OF THE DISCLOSURE

The present disclosure relates to methods and systems for determining a physical characteristic of a medium. In particular, the method is suitable for providing image data of a medium scanned by a transducer device. For example, the method may be used in a device such as for instance an ultrasound system, that may be a medical device.

BACKGROUND OF THE DISCLOSURE

Generally, transducer elements or transceivers (for example arranged as an array) may serve for different purposes, for example for communication, imaging or scanning (for example in the field of medical imaging), radar, sonar, seismology, wireless communications, radio astronomy, acoustics and biomedicine. One example comprises ultrasound imaging.

In a conventional ultrasound imaging method, an ultrasound transducer device (also referred to as an ultrasound probe) with a set of ultrasound transducer elements may be used to generate ultrasound pulses that insonify a medium of interest in a transmission operation. The medium may comprise one or several tissues such as for example fat, muscles, bones, and blood vessels. Then, in a reception operation a set of echo signals is received from the medium by the set of transducer elements. In particular, each of the transducer elements converts a received echo signal into for example an electrical signal. The signal may further be processed by an ultrasound system. For example, they may be digitalized, demodulated, filtered, and/or a signal conditioning operation may be carried out.

Before insonification (i.e. ultrasound excitations sent to the medium) a contrast agent (for example small air bubbles) may be introduced into the medium. In particular, in the application of Contrast-Enhanced ultrasound (CEUS), the medium is insonified following a dedicated transmission sequence (i.e. ensemble of transmitted pulses) to better image the ultrasound contrast agents.

The contrast agents may be injected into the vascular system of the patient and imaged during a contrast ultrasound scan. The goal of this technique is to analyse the bubble behaviour, notably within and around suspicious tumour(s).

To better observe the contrast agent, pulse modulation techniques have been proposed to distinguish between tissues and contrast agent. These techniques take advantages of the non-linearity of the bubble response compared to the linear response of the tissue. In particular, CEUS imaging relies on the non-linear response of the ultrasound contrast agents (compare to the linear response of soft tissues). By means of an amplitude and/or phase modulation, backscattered signals generated by ultrasound contrast agents are separated from the echoes generated by tissues. Such process requires to successively insonify the medium with transmitted pulses associated with different amplitude and/or phase cf. e.g.:

Tremblay-Darveau C, Sheeran PS, Vu CK, et al. The Role of Microbubble Echo Phase Lag in Multipulse Contrast-Enhanced Ultrasound Imaging. IEEE Trans Ultrason Ferroelectr Freq Control. 2018; 65(8):1389-1401. doi:10.1109/TUFFC.2018.2841848

Furthermore, EP3097432A1 describes a method for determining a physical characteristic on a punctual location inside a medium, comprising the steps of: introducing a contrast agent into the medium, sending an emitted sequence comprising emitted pulses having different amplitudes, receiving a received sequence comprising received pulses corresponding to echoes of said emitted pulses, calculating a phase difference between the received pulses relative to the emitted pulses, and determining the physical characteristic on the bases of said phase difference.

SUMMARY OF THE DISCLOSURE

Currently, it remains desirable to provide a method and system for determining a physical characteristic of a medium which can further enhance the quality of the determined characteristic. In particular, it remains desirable to more effectively filter the information of interest in the signal data of the medium, for example image data of an introduced contrast agent. Furthermore, it is desirable to reduce noise in the signal data of the medium, and/or to better filter and/or cancel a signal generated by soft tissues in CEUS imaging mode.

Therefore, according to the embodiments of the present disclosure relates to a method for determining a physical characteristic of a medium, comprising:

- processing (d) ultrasound signal data of the medium associated to at least three emitted ultrasound pulses for respectively providing at least three in-phase and quadrature phase (IQ) data sets $I_1(r)$, $I_2(r)$, $I_3(r)$, the at least three emitted ultrasound pulses comprising a first emitted pulse having a first intensity and at least two supplementary emitted pulses having each a second intensity, wherein a sum of the second intensities corresponds to the first intensity,
- determining (e) the physical characteristic as a function of:

  a first phase lag between the first IQ data set $I_1(r)$ and a sum of the at least two further IQ data sets $I_2(r), I_3(r)$, and/or
  a second phase lag between the at least two further IQ data sets $I_2(r)$, $I_3(r)$.

By providing such a method, it becomes possible to enhance the quality of the determined characteristic.

In particular, by determining (e) the physical characteristic as a function of a first phase lag between the first IQ data set $I_1(r)$ and a sum of the at least two further IQ data sets $I_2(r)$, $I_3(r)$, it becomes possible to detect more effectively an information of interest in the ultrasound signal data, for example image data of an introduced contrast agent.

Then, by determining (e) the physical characteristic as a function of a second phase lag between the at least two further IQ data sets it becomes also possible to detect noise in the ultrasound signal data.

As a further consequence, undesired data in the ultrasound signal data may be determined by excluding the detected information of interest and the detected noise. In other words, everything that is not detected as an information of interest or as noise, may be classified as undesired data. Such undesired data may in particular comprise ultrasound signal data of a tissue present in the medium.

Accordingly, the method may further comprise classifying the determined physical characteristic (or a value of a map of physical characteristics as described below) into one of three categories: information of interest, noise and undesired data (for example tissue).

For example, a determined physical characteristic classified as information of interest may be emphasized, meanwhile any determined physical characteristic which has been classified as noise or undesired data may be suppressed or cancelled. Accordingly, a determined physical characteristic classified as information of interest may be better distinguished from any determined physical characteristic which has been classified as noise or undesired data.

Detecting information of interest and/or noise may comprise at least one of characterizing, recognizing, emphasizing, highlighting, or generally becoming aware of information of interest and/or noise.

The ultrasound signal data may be processed for providing (or obtaining) the complex IQ data sets. Accordingly, this may mean that each IQ data set is respectively associated to an emitted ultrasound pulse.

The at least two supplementary emitted pulses may have the same (second) intensity and/or the same amplitude.

The sum of amplitudes (for example the peak values) of the supplementary emitted pulses may correspond to an amplitude (for example the peak value) of the first emitted pulse.

The IQ data sets may be demodulated IQ ultrasound data sets and/or IQ beamformed data sets.

More generally an IQ data set may comprise IQ data. For example, an IQ data set may comprise at least one pair of an in-phase value and a quadrature phase value. Accordingly, the IQ data may be demodulated IQ ultrasound data and/or IQ beamformed data.

Determining (e) the physical characteristic may further comprise setting the first IQ data set $I_1(r)$ in-phase with the sum of the at least two further IQ data sets $I_2(r)$, $I_3(r)$ by introducing a compensation phase lag to the first IQ data set $I_1(r)$ and/or the sum of the at least two further IQ data sets $I_2(r)$, $I_3(r)$. In other words, the additional phase lag may be selected such that a phase lag between the $I_1(r)$ and a sum of $I_2(r)$, $I_3(r)$ is reduced and/or compensated. Accordingly, an optimal coherent summation may advantageously be obtained.

Determining (e) the physical characteristic may further comprise: setting the first IQ data set $I_1(r)$ in opposite phase to the sum of the at least two further IQ data sets $I_2(r)$, $I_3(r)$ by introducing a phase shift of $\pi$ to the first IQ data set $I_1(r)$, optionally subtracted by the compensation phase lag (i.e.: $(\pi - \phi_1(\mathbf{r}))$, as cited in the equation (1d) below). This phase shift can advantageously lead to an improved noise reduction in the determined physical characteristic.

The first phase lag $\phi_1(\mathbf{r})$ between the first IQ data set $I_1(r)$ and a sum of the at least two further IQ data sets $I_2(r)$, $I_3(r)$ may be measured by (or may correspond to):

  a difference between the phase of $I_1(r)$ and the phase of the sum of $I_2(r)$, $I_3(r)$, or
  the phase of the scalar product between $I_1(r)$ and a sum of $I_2(r)$, $I_3(r)$, i.e. expressed as an equation by:

$$\phi_1(\mathbf{r}) = \arg[I_1(\mathbf{r}) \times \mathrm{conj}(I_2(\mathbf{r}) + I_3(\mathbf{r}))] \ (2)$$

The compensation phase lag may be determined as a function of $\phi_1(r)$. In order to obtain a less aggressive compensation, it is also possible to only partially (i.e. not fully) compensate $\phi_1$. In other words, it may be of interest to insert a compensation phase lag so that the first IQ data set $I_1(r)$ and the sum of the at least two further IQ data sets $I_2(r)$, $I_3(r)$ may become more in phase or in opposite phase (optionally without becoming perfectly in phase or in opposite phase).

The second phase lag $\phi_2(\mathbf{r})$ between the at least two further IQ data sets $I_2(r)$ and $I_3(r)$ may be measured by (or may correspond to):

> a difference between the phase of $I_2(r)$ and the phase of $I_3(r)$, or
> a phase of the scalar product of $I_2(r)$ and $I_3(r)$, which may in particular be expressed by:

$$\phi_2(\mathbf{r}) = \arg\left[I_2(\boldsymbol{r}) \times \mathrm{conj}\left(I_3(\boldsymbol{r})\right)\right] \qquad (4).$$

Determining(e) the physical characteristic may further comprise determining a first segmentation map as a function of the first phase lag and/or a second segmentation map as a function of the second phase lag. The physical characteristic may then be determined as a function of the first and/or the second segmentation map.

The physical characteristic may be determined as a function of a third segmentation map generated by combining the first segmentation map and the second segmentation map. For example, the third segmentation map may be generated by removing the content of the second segmentation map from the first segmentation map.

Accordingly, the quality of the determined characteristic (and optionally of image data formed based on said characteristic) may be enhanced, in particular by enhancing IQ data of a region of interest in the medium and by reducing noise and undesired data (e.g. tissue signals).

The first segmentation map may be configured to segment an area of interest of the medium. For example, the region of interest may be an area where the medium comprises a contrast agent (as explained below). The first segmentation map may be used for example to capture (or highlight) said area of interest, that may be shown/displayed in a formed image.

A map may be understood as a matrix or a table configured to assign data values to data fields, for example values of physical characteristics to regions in the medium. It may be of 2D, or more dimensions, and number of columns and lines may be equal or different. In one example, it may comprise a plurality of pixels, for example forming an image.

However, a segmentation map may also comprise only one value. In this case the segmentations maps may serve as global filters. For example, in case the first and/or second segmentation map determines that physical characteristic is zero, a corresponding output may be generated and may be provided to a user or a processing system. For example, said output may indicate that the ultrasound signal data are not suitable for determining a physical characteristic of the medium (for example due to too much noise or an insufficient data quality). In this case, the method may be repeated, or a repletion may be suggested.

It is further noted that a segmentation map may be used as a binary filter. In other words, it may define for each value, whether it is used or disregarded. However, a segmentation map may also be used as a soft and/or spatial filter. For example, it may assign each value with a weighting (for example between 0 and 1).

Moreover, with regard to the determined compensation phase lag, also a smooth compensation method may be applied using for example a spatial and/or temporal filter.

The second segmentation map may be configured to detect and/or reduce undesired signals and/or noise in the IQ data sets $I_1(r)$, $I_2(r)$, $I_3(r)$. For example, the noise may origin from the medium and/or from electronic noise.

The first segmentation map may be determined by:

$$\phi_1^{\mathrm{seg}}(\mathbf{r}) = \begin{pmatrix} 1, \ \text{if} & \phi_1^{\min} < \phi_1(\mathrm{r}) < \phi_1^{\max} \\ 0, \ \text{else} \end{pmatrix} \qquad (3),$$

wherein $\phi_1^{\min}$ and $\phi_1^{\max}$ are predefined values. Generally, the values may be defined such that a region of interest in the medium is captured and any other parts are excluded. Accordingly, $\phi_1^{\min}$ and $\phi_1^{\max}$ may be predefined as a function of the phase lag between IQ data associated to the region of interest and of other regions. Exemplary values may comprise $\phi_1^{\min} = -2.5$ rad and/or $\phi_1^{\max} = -0.5$ rad. However, the values may also differ. For example $\phi_1^{\min}$ may also be defined by a range between for example -3 and -2 rad, and/or $\phi_1^{\max}$ may also be defined by a range between for example -1 and 0 rad. Also in these cases it can be assured that the information of interest can be sufficiently detected, i.e. sufficiently distinguished from undesired data (for example from tissue).

The second segmentation map may be determined by:

$$\phi_2^{seg}(r) = \left(\begin{matrix} 1, if & |\phi_2(r)| > \phi_2^{max} \\ 0, else \end{matrix}\right) (5),$$

wherein $\phi_2^{max}$ is a predefined value. Generally, the value may be defined such that it highlights the noise in the IQ data. Accordingly, $\phi_2^{max}$ may be predefined as a function of the phase lag between IQ data associated to regions generating noise and of other regions. An exemplary value may comprise $\phi_2^{max} = 0.5$ rad. However, the value may also differ. For example $\phi_2^{max}$ may also be defined by a range between for example 0.4 and 0.6 rad or more broadly between 0.25 and 1 rad. Also in these cases it can be assured that noise can be sufficiently distinguished from the information of interest.

The third segmentation map may be determined by:

$$\phi_3^{seg}(r) = \left(\begin{matrix} 1, & \text{if} & \phi_1^{seg}(r) = 1 \text{ and } \phi_2^{seg}(r) = 0 \\ 0, & \text{else} \end{matrix}\right) \quad (6).$$

The physical characteristic may be determined based on at least one of:

a predefined contrast-enhanced ultrasound method,
a coherent summation of the IQ data sets $I_1(r)$, $I_2(r)$, $I_3(r)$,
the equation (1b):

$$I_{ceus}^+(r) = (I_1(r) - (I_2(r) + I_3(r))) \times \phi_3^{seg}(r),$$

the equation (1c):

$$I_{ceus}^{++}(r) = I_1(r) \times e^{-i\,\phi_1(r) \times \phi_3^{seg}(r)} - (I_2(r) + I_3(r)),$$

the equation (1d):

$$I_{ceus}^-(r) = I_1(r) \times e^{-i\,(\pi - \phi_1(r)) \times \phi_2^{seg}(r)} - (I_2(r) + I_3(r)),$$

the equation (1e):

$$I_{ceus}^{--}(r) = I_1(r) \times e^{-i\,(\pi - \phi_1(r)) \times (1 - \phi_3^{seg}(r))} - (I_2(r) + I_3(r))$$

the equation (1f):

$$I_{ceus}^{++-}(r) = I_1(r) \times e^{-i\,\phi_1(r) \times \phi_3^{seg}(r)} \times e^{-i\,(\pi - \phi_1(r)) \times \phi_2^{seg}(r)} - (I_2(r) + I_3(r)),$$

and the equation (1g):

$$I_{ceus}^{++--}(\mathbf{r}) =$$

$$I_1(\mathbf{r}) \times e^{-i\,\phi_1(\mathbf{r}) \times \phi_3^{seg}(\mathbf{r})} \times e^{-i\,(\pi - \phi_1(\mathbf{r})) \times (1 - \phi_3^{seg}(\mathbf{r}))} - \big(I_2(\mathbf{r}) + I_3(\mathbf{r})\big).$$

The method may further comprise, before processing the ultrasound signal data:

transmitting (b) an emitted sequence of ultrasound waves into the medium (11), the emitted sequence comprising the first emitted pulse and the at least two supplementary emitted pulses, and
receiving (c) a response sequence of ultrasound waves from the medium, wherein the ultrasound signal data are based on the response sequence of ultrasound waves.

The transmission and reception may be carried out by the same or different transducer elements, or even by different transducer devices.

The at least two supplementary emitted pulses may for example spatially differ from each other with respect to the medium, in particular with respect to the areas insonified by the pulses. In other words, the first one of the two supplementary pulses may insonify a different area of the medium than the second one. However, the respectively insonified areas may predominantly overlap. For example, the supplementary emitted pulses may be generated by different transducer elements of a transducer device.

It is also possible that the supplementary emitted pulses are emitted with different aperture modulations. For example, a transducer device used for emitting the pulses may have an adjustable aperture. Said aperture may then be modulated in different ways for the second and third pulse.

The emitted sequence may be sent using an ultrasound transducer device comprising a plurality of ultrasound transducer elements.

A set of transducer elements used for emitting the first pulse may be split into at least two sub-sets which are respectively used to emit the at least two supplementary pulses. For example, a first sub-set may correspond to the ODD numbers of the transducer elements used for emitting the first supplementary pulse, and a second sub-set may correspond to the EVEN numbers of the transducer elements used for emitting the second supplementary pulse.

Accordingly, by using different sub-sets of transducer elements for the different supplementary pulse, the effective aperture of the transducer device can be modulated for each pulse.

It is also possible to use other configurations of transducer elements for the second and the third pulse than the afore-mentioned EVEN and ODD pulses. For example, any configuration may be used, which equally distributes the number of transducer elements used for the first pulse between the further pulses (i.e. the second and third pulse and optionally any supplementary pulse). In other words, the set of transducer elements used for emitting the first pulse may be split into at least two (or more) equal sub-sets which are respectively used to emit the at least two (or more) supplementary pulses.

For example, a (or each) sub-set may also comprise at least one group of consecutive transducer elements (for example 2-10). The transducer elements of a (or each) group may hence be consecutively arranged in the transducer device (i.e. next to each other). In case the (or each) sub-set comprises more than one group, the groups of different sub-sets (i.e. associated with different supplementary pulses) may be alternating.

The first pulse may have an inverse polarity compared to the pulse of second intensity. For example, with reference to equation (1b), in case an inverse polarity is used, the equation may be adapted by introduction of $\varepsilon$ to equation (1b'):

$$I_{ceus}^{+}(\mathbf{r}) = (I_1(\mathbf{r}) - (\epsilon)\big(I_2(\mathbf{r}) + I_3(\mathbf{r})\big)) \times \phi_3^{seg}(\mathbf{r})$$

where $\varepsilon$ = -1. Also the further equations (1c) to 1g) may be adapted correspondingly.

In one example, the first emitted pulse may be generated using a first number of transducer elements, and each of the supplementary emitted pulses may be generated using a second number of transducer elements. The first number may correspond to the second number multiplied by the number of supplementary emitted pulses.

The response sequence may be a set of backscattered signals. For example, the transducer elements may receive backscattered signals from the medium and transform them into for example electrical signals.

For example, an emitted pulse may comprise insonification of the medium with a cylindrical wave that focuses on a given point and/or with a plane wave and/or with a diverging wave. The response sequence may comprise the backscattered echoes of this insonification.

More in particular, a plurality of ultrasonic waves may be transmitted into a region of interest and in response a set of raw data may be acquired by a set of transducer elements in response to each ultrasonic wave, the ultrasonic waves having different spatial frequency content.

The received set of signals may be a set of ultrasound signals.

The transducer device may be an ultrasound transducer device. The transducer elements may be for example in the form of a transducer array, for example a 1D transducer array (having a line of transducer elements), 1.5D transducer array (having one or two lines of transmission transducer elements and one or two lines of reception transducer elements) or 2D or a matrix transducer array (having multiple lines of transducer elements). However, the transducer device is not limited to a transducer element of an ultrasound system. Instead, the transducer device may emit and receive any kind of waves; Further examples comprise a transducer device of a radar system, a sonar system, a seismology system, a wireless communications system, a radio astronomy system, an acoustics system, a Non Destructive Testing (NDT) system, and/or a biomedicine system or the like.

The method may further comprise before sending the emitted sequence of ultrasound waves:

introducing (a) a contrast agent into the medium,
wherein the first segmentation map is configured to segment the contrast agent in the medium.

The present disclosure may further refer to a method for determining physical characteristics of a plurality of regions of a medium, comprising repeatedly performing the method according to any one of the preceding claims, wherein in each iteration ultrasound signal data of another region is processed.

Accordingly, also an emitted sequence of ultrasound waves may be transmitted into each of the plurality of regions, in order to receive for each region a response sequence.

Therefore, a map of physical characteristics may be determined which comprises a plurality of physical characteristic values, for example one value for each region. In one example, the map of physical characteristics may comprise a plurality of pixels, for example for forming an image. In other words, each physical characteristic values may be represented by a pixel.

At least one of the first, second and third segmentation map may be determined as a function of the plurality of regions. Accordingly, the maps may have resolutions (or number of values) adapted to the number of regions.

An image may be formed based on the determined physical characteristics of the plurality of regions.

The present disclosure further refers to a computer program comprising computer-readable instructions which when executed by a data processing system cause the data processing system to carry out the method according to any one of preceding method claims.

The present disclosure may also relate to a recording medium readable by a computer and having recorded thereon a computer program including instructions for executing the method according to the present disclosure, when said program is executed by a computer.

The present disclosure further refers to a system for determining a physical characteristic of a medium comprising a processing unit configured to: process ultrasound signal data of the medium associated to at least three emitted ultrasound pulses for respectively providing at least three in-phase and quadrature phase (IQ) data sets $I_1(r)$, $I_2(r)$, $I_3(r)$, the three emitted ultrasound pulses comprising a first emitted pulse having a first intensity and at least two supplementary emitted pulses having each a second intensity, wherein a sum of the second intensities corresponds to the first intensity, determine the physical characteristic as a function of:

a first phase lag between the first IQ data set $I_1(r)$ and a sum of the at least two further IQ data sets $I_2(r)$, $I_3(r)$, and/or
a function of a second phase lag between the at least two further IQ data sets $I_2(r)$, $I_3(r)$.

The system may comprise the transducer device. The system may comprise in particular a probe (for example an ultrasound probe) which may comprise the transducer device.

The system may be an ultrasound system.

The system may comprise further functional characteristics and/or may be configured in correspondence to the method operations described above.

The disclosure and its embodiments may be used in the context of medical systems dedicated to human beings, plants or animals but also any (non-living) soft material to be considered.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, are provided for illustration purposes, and illustrate embodiments of the disclosure and together with the description and serve to support and illustrate the principles thereof.

BRIEF DESCRIPTION OF THE DRAWINGS

Fig. 1 shows an exemplary embodiment of the method according to embodiments of the present disclosure;
Fig. 2 shows a system carrying out a method according anexemplary embodiment of the present disclosure;
Fig. 3 shows a system carrying out a method according to anotherexemplary embodiment of the present disclosure;
Fig. 4 schematically shows different types of emitted pulses according to embodiments of the present disclosure;
Fig. 5 schematically shows the procedure of determining the first segmentation map according to the present disclosure; and
Fig. 6 schematically shows the procedure of determining the second segmentation map according to the present disclosure.

DESCRIPTION OF THE EMBODIMENTS

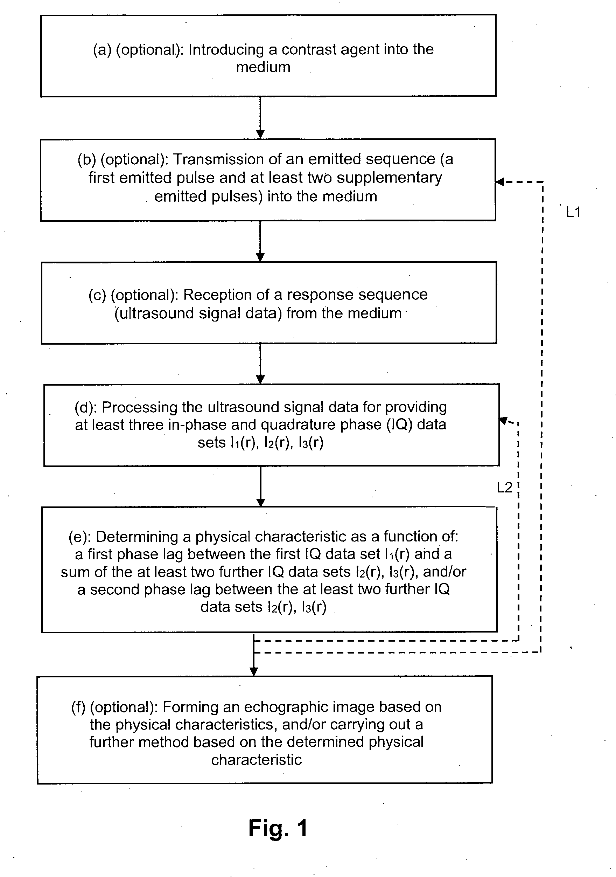

Reference will now be made in detail to exemplary embodiments of the disclosure, examples of which are illustrated in the accompanying drawings. Wherever possible, the same reference numbers will be used throughout the drawings to refer to the same or like parts. Moreover, the features explained in context of a specific embodiment, for example that one of fig. 1, also apply to any one of the other embodiments, when appropriate, unless differently described.

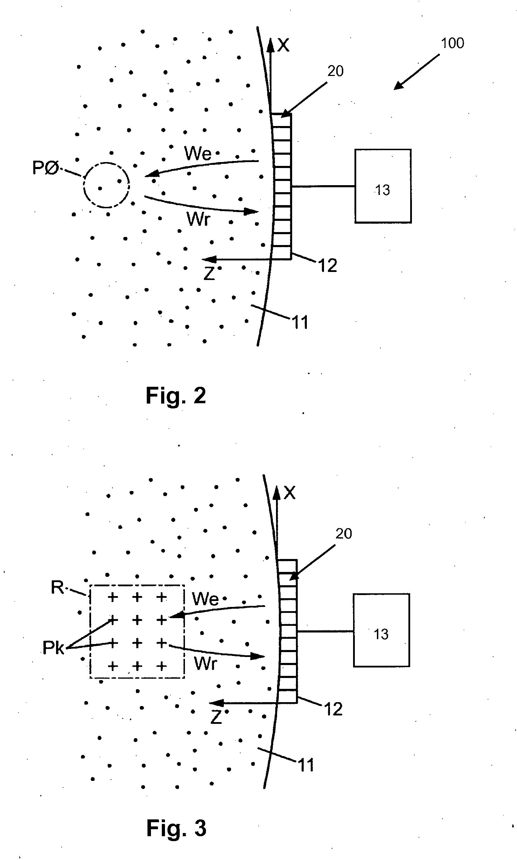

Fig. 1 shows an exemplary embodiment of the method according to embodiments of the present disclosure. The method may be carried out by means of a system 1, more in particular by an ultrasound system 20. Examples are described in context of figures 2 and 3.

The method may be an ultrasound method carried out by an ultrasound system. Possible ultrasound methods comprise B-mode imaging, shear wave elastography imaging (such as ShearWave® mode developed by the applicant, Doppler imaging, M mode imaging, ultrafast™ Doppler imaging or angio mode named under Angio P.L.U.S ™ ultrasound imaging or any other ultrasound imaging mode, including a mode using bubbles and/or a mode using contrast agent(s) or the like. The method may be part of any of the above-mentioned methods or may be combined with any of these methods.

However, the method according to the present disclosure may also be applied to other technical fields than ultrasound examination. In particular, any technical field is possible which uses a plurality of transducer elements to acquire data/signals of an examined medium or environment and/or which may optionally use a beamforming technique based on the collected data/signals. Examples comprise methods using a radar system, sonar system, seismology system, wireless communications system, radio astronomy system, acoustics system, Non-Destructive Testing (NDT) system and biomedicine system. The principle of emitting pulses of different intensity by respectively selecting different numbers of transducer elements of an ensemble of transducer elements (e.g. all transducer elements, or only ODD/EVEN ones), what would conventionally lead to a corresponding number of channels, is always similar.

Accordingly, the method according to the present disclosure may in each of these cases achieve the same positive technical effects as described above, for example of enhanced quality of the determined physical characteristic. However, for mere illustration purposes of the present disclosure, in the following it is referred to the example of an ultrasound method.

The method may be for example a Contrast-enhanced ultrasound (CEUS) method. In the CEUS method, a contrast agent is provided in a region of interest of the medium (cf. operation (a)), which is then scanned (cf. operations (b), (c)).

In more detail, in an optional operation (a), a contrast agent is introduced (or injected) into the medium. Said contrast agent may comprise for example micro bubbles. The contrast agent may be configured to improve the contrast of physical properties of a region of interest of the medium. When insonified by ultrasound waves, such contrast agents generate non-linear echoes. Said operation (a) may be carried out by an injection device (not shown in the figures). The injection device may be configured to inject a fluid containing a predetermined quantity of contrast agents inside blood vessels of the medium.

In an optional operation (b) an emitted sequence of ultrasound waves is transmitted into the medium. The emitted sequence comprises a first emitted pulse and at least two supplementary emitted pulses. The first emitted pulse may have a first intensity and at least two supplementary emitted pulses may have each a second intensity, wherein a sum of the second intensities corresponds to the first intensity. For example, transmitting a pulse may comprise insonification of the medium with a cylindrical wave that focuses on a given point and/or plane waves of different angles. More in particular, transmitting a pulse may comprise transmitting a plurality of ultrasonic waves into an imaged region.

In an optional operation (c) a response sequence of ultrasound waves is received from the medium. Said response sequence may form the basis or may correspond to ultrasound signal data which will be processed in the next operation (d). For example, in the operation (c) backscattered echoes of the insonification of operation (b) may be used. More in particular, in the operation (c) a set of raw data may be acquired by a set of transducer elements in response to each emitted pulse.

It is noted that operations (a) to (c) are optional, as they may also be carried out by any other system than the system used for operations (d) and (e). Data may also be provided by other functionalities such as simulation devices, insonification on a phantom, etc. It is also possible that the ultrasound signal data processed in operation (d) are pre-stored, and for example provided by a data storage, a communication interface, etc.

In operation (d) ultrasound signal data of the medium associated to at least three emitted ultrasound pulses are processed for respectively providing (or obtaining) at least three in-phase and quadrature phase (IQ) data sets $I_1(r)$, $I_2(r)$, $I_3(r)$. The three emitted ultrasound pulses may comprise a first emitted pulse having a first intensity and at least two supplementary emitted pulses having each a second intensity, wherein a sum of the second intensities corresponds to the first intensity. However, it is also possible that more than three emitted ultrasound pulses are processed (e.g. 4) for respectively providing more than three IQ data sets (e.g. 4).

In operation (e) the physical characteristic as a function of a first phase lag between the first IQ data set $I_1(r)$ and a sum of the at least two further IQ data sets $I_2(r)$, $I_3(r)$, and/or a second phase lag between the at least two further IQ data sets $I_2(r)$, $I_3(r)$.

In an optional operation (f), an echographic image based on the physical characteristics, and/or carrying out a further method based on the determined physical characteristic is generated. For example, the determined physical characteristic may be used in a further algorithm (for example an AI-based algorithm) to determine or predict a diagnosis of the medium.

The operations (a) to (e) may be carried out successively, i.e. one after another. However, operations (b) to (e) and/or (d) to (e) may be carried out several times before thanks to optional loops L1 or L2. Hence, operation (f) may be carried out based on the respective plurality of determined characteristics. For example, in each iteration, another area in the medium may be insonified in operation (b), in order to determine respective plurality of determined characteristics associated to said different regions. Said determined characteristics may then serve to form a map, matrix or table of determined characteristics, and/or to form an image to be potentially displayed and/or analyzed , right after or in the future, locally or remotely.

The operations (b) and (c) may be carried out by a transducer device 12, for example mounted on an ultrasound probe. The transducer device is desirably a hand-held system. The output of the system 1 may be transmitted via an interface 10 to a central or main or external processing system having a processing unit 13 (cf. fig. 2 and 3).

The operations (d) and (e) may be carried out by a processing unit 13. Operation (f) may be carried out by a display or screen 4a associated with the processing system 4.

Fig. 2 shows a system carrying out a method according to an exemplary embodiment of the present disclosure.

The system 100 may for example be configured to determine a characteristic of a location inside a medium 11, or for instance for the purpose of imaging an area in a medium 11.

The medium 11 is for instance a living body and in particular human or animal bodies, or can be any other biological or physic-chemical medium (e.g. in vitro medium). The medium may comprise variations in its physical properties. For example, the medium may comprise tissues such as fat, muscles, bones, and blood vessels, each one having various physical properties.

For example, the tissue may comprise an area suffering from a disfunction and/or an illness (e.g. cancerous cells, muscular tearing, ...), or any other singular area, having various physical properties in comparison to other area of the medium. Some portions of the medium 11 may include some added contrast agent (e.g. micro bubbles) for improving the contrast of physical properties of these portions. When insonified by ultrasound waves, such contrast agents generate non-linear echoes. Therefore, a possible use of such contrast agents is the injection of a fluid containing a predetermined quantity of contrast agents inside blood vessels or dedicated part(s) of the body. The contrast agent may however also be introduced into the medium in another way. It may for example be injected into other parts or circulation systems of a body than a blood vessel. Moreover, it may also be inhaled or swallowed. Then, the physical characteristic of such blood vessels can be more easily detected in comparison to a physical characteristic of a tissue that does not comprise the contrast agent, as said contrast agent may only flows in the vessels.

The physical characteristics, that can be detected by the method that senses the medium via ultrasound waves, may be mechanical properties of the medium, like stiffness, or else. The method distinguishes values and/or variations of said physical properties. For example, the method may detect a mechanical interface between two materials in the medium. For example, it can detect bubble shells. Ultrasound contrast agents namely generally rely on the different ways in which sound waves are reflected from interfaces between substances. This may be the surface (i.e. the shell) of a small air bubble or a more complex structure.

The system 100 may include a probe 12 comprising a transducer device. Said transducer device may comprise one or a plurality of ultrasound transducer elements 20, for example in the form of a transducer array arranged along an x-axis. Each transducer element 20 may be adapted to transform a signal into an ultrasound wave (emit) and/or to transform an ultrasound wave into a signal (receive).

The system 100 may further include an electronic processing unit 13. Said unit may optionally control the transducers in the probe in both mode (receive and/or emit) in the case same probe is used for emission/reception. Different probes may also be used, either for emission/reception or for appropriate adaptation to scanned medium. Emit and receive transducers may be the same, different one located on one single probe or on different probes.

Furthermore, the unit 13 may process ultrasound signal data, and determine characteristics of the medium and/or images of said characteristics.

The probe 12 may comprise a curved transducer so as to perform an ultrasound focusing to a predetermined position in front of the probe into a direction of a z axis. The probe 12 may also comprise a linear array of transducer. Moreover, the probe 12 may comprise few tens of transducer elements (for instance 124, 258, or 64 to 300) juxtaposed along an x axis so as to perform ultrasound focusing into a bi-dimensional (2D) plane. The probe 12 may comprise a bi-dimensional array so as to perform ultrasound focusing into a tri-dimensional (3D) volume. Moreover, the probe may also comprise several transducer devices, for example at least one for emission and at least one for reception.

A first configuration of the method represented on figure 1 is for determining a physical characteristic of a location P0 inside the medium 11, said location P0 being substantially a punctual location or a small region inside the medium around said location P0 (near location P0).

The above processing unit 13 and the probe 12 may be configured to send an emitted sequence ES of ultrasound waves We into the medium 11 towards the location P0, the emitted sequence ES comprising at least three emitted pulses. Those three emitted ultrasound pulses may comprise a first emitted pulse having a first intensity and at least two supplementary emitted pulses having each a second intensity, wherein a sum of the second intensities corresponds to the first intensity. The above processing unit 13 and the probe 12 may further be configured to receive a received sequence RS of ultrasound waves 4 (i.e. ultrasound signal data) from the location P0 in response to the emitted pulses.

The ultrasound waves We, Wr toward and from the location may be a focused wave (beam) or a non-focused beam. In this context, a pre-defined beamforming method may be used, for example: The emitted ultrasound wave We may be generated by a plurality of transducers signals that are delayed and transmitted to each transducer of a transducer array. The received ultrasound wave Wr may be composed of a plurality of transducers signals that are combined by delay and summation to produce a received sequence RS.

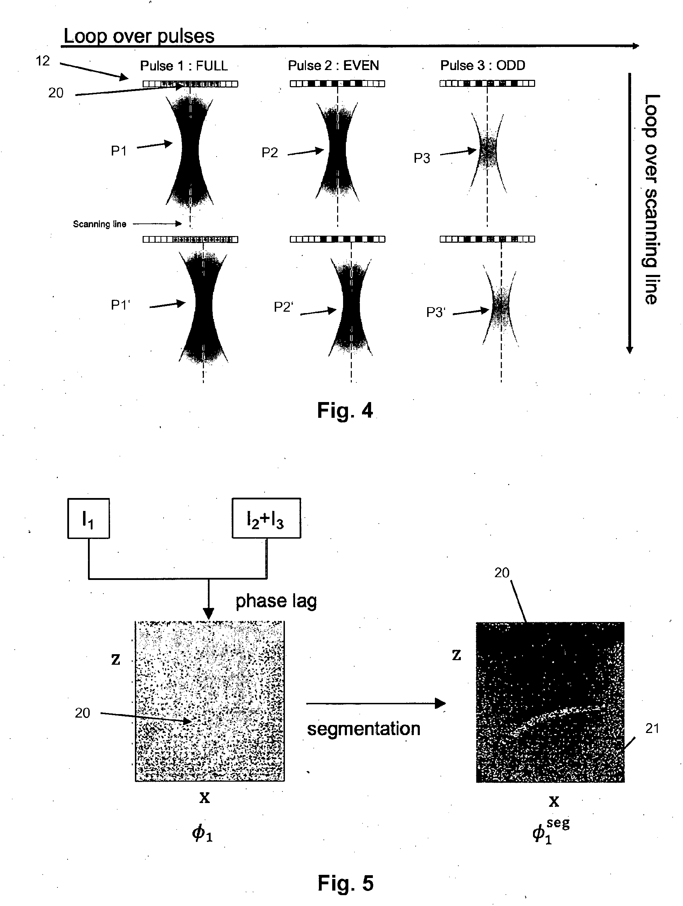

The at least two different intensities or amplitudes of emitted pulses (i.e. the first pulse having a first intensity and the second and third pulse having each the same second intensity) may be produced by varying the transmit voltage or by varying the aperture size (i.e. by varying the number of transducers elements contributing to emit the emitted ultrasound wave, as further described in context of fig. 4). The aperture may also be divided into two or more groups of elements.

Fig. 3 shows a system carrying out a method according to another exemplary embodiment of the present disclosure.

The second exemplary embodiment may generally correspond the first exemplary embodiment. In particular, the method may use identical or similar elements of the above disclosed system 100 of figure 2.

However, in the second exemplary embodiment of the method may determine physical characteristics of a plurality of regions of the medium 11. For example, the determined physical characteristics may be used for determining an image of a region R inside a medium 11.

The image produced by the method may be composed of a plurality of pixels (for example, a number K of pixels), each pixel corresponding to a different location (Pk) inside the region R, k being an index to identify each pixel in the image to be generated or each location in the region R. Optionally, the image may be composed of only one pixel. However, the image may comprise more than one ten thousand pixels (100x100 pixels).

Moreover, it is noted that the determined physical characteristics may also be used for other purposes than forming an image. For example, the determined physical characteristics may be processed in another method to determine further characteristics and/or a diagnosis about the region R. The determined physical characteristics may also be transferred to an AI system or function, to train it, and/or to support diagnosis and/or to provide inputs to another system and/or display info to an end user.

The second embodiment (shown in fig. 3) mainly differs from the previous first embodiment of the method by scanning a plurality of locations inside a region R so as to generate for example an image of said region.

At each location Pk inside the region R, the processing unit 13 and the probe 12 are configured to send an emitted sequence ES of ultrasound waves We towards the location, the emitted sequence ES comprising at least three emitted pulses, said pulses having different intensities, and receive a received sequence RS of ultrasound waves Wr from the location, said received pulses being responses (echoes) from said emitted pulses.

Similarly, as for the previous embodiment, the ultrasound waves We, can be focused or non-focused waves, according to known techniques. The emitted and received signals (representing the pulses) may also be similar or identical to those as represented on figure 4, and the corresponding above description also applies to the second configuration of the method.

Fig. 4 schematically shows different types of emitted pulses according to embodiments of the present disclosure. In particular, fig. 4 shows three emitted ultrasound pulses P1, P2, P3. The first emitted pulse P1 has a first intensity and the two supplementary emitted pulses P2, P3 have each a second intensity, wherein a sum of the second intensities corresponds to the first intensity. The first intensity is hence double the second intensity in this example.

The different intensities in this example are due to the number of transducer elements 20 used to generate the pulse. Accordingly, the number of transducer elements used for emitting the first pulse may correspond to the total number of transducer elements for emitting all supplementary pulses. More in particular, the first pulse may be generated using a predefined number of adjacent transducer elements 20 (i.e. a "full" pulse). The second pulse may be generated using the EVEN numbered transducer elements 20 (i.e. an "EVEN" pulse) and the third pulse using the ODD numbered transducer elements 20 (i.e. an "ODD" pulse). ODD and EVEN pulses may also be vice versa.

It is also possible to use other configurations of transducer elements 20 for the second and the third pulse the EVEN and ODD pulses. For example, any configuration may be used, which equally distributes the number of transducer elements used for the first pulse between the further pulses (i.e. the second and third pulse and optionally any supplementary pulse). In other words, the set of transducer elements used for emitting the first pulse may be split into at least two (or more) equal sub-sets which are respectively used to emit the at least two (or more) supplementary pulses.

For example, a sub-set may also comprise at least one group of consecutive transducer elements (for example 2-10). The transducer elements of a group may hence be consecutively arranged in the transducer device (i.e. next to each other). In case the sub-set comprises more than one group, the groups of different sub-sets (i.e. associated with different supplementary pulses) may be alternating.

The pulses may be looped over time, i.e. first the pulse P1 may be emitted, then P2, then P3. This sequence may be repeated. In particular, the sequence may be repeated by looping over a set scanning lines. In other words, the scanning line or focus of the emitted pulses may be offset in an x-direction (cf. fig. 2) at each iteration. Accordingly, after emission of P1, P2, P3, the method may continue with the emission of spatially offset P1', P2', P3'. Apart from the mentioned offset, P1', P2', P3' may correspond to P1, P2, P3.

It is further possible that the focal point along the scanning line is offset in a depth direction of the medium in a further loop (i.e. along the z-axis in fig. 2). In this way different regions of the medium can be scanned, wherein each region is insonified by the at least three pulses.

Accordingly, based on these pulses, a map (or matrix or table) of determined physical characteristics of the medium may be formed. Said map may serve to image the medium and/or to feed another method, e.g. an (AI-based) algorithm, for example comprising a machine learning algorithm, in particular one or several neural networks.

Said three pulses P1, P2, P3, in particular comprising a "full" pulse, an "EVEN" pulse and an "ODD" pulse may be used in a Contrast-enhanced ultrasound" (CEUS) method.

Concerning the CEUS mode, contrast agent may be injected for example in the vascular system of a patient and corresponding region(s) of interest scanned during a contrast ultrasound scan. The goal of this technique is to analyze the contrast agent behavior, notably within and around suspicious tumors. To better observe the contrast agent, pulse amplitude and/or phase modulation techniques may be applied to advantageously distinguish between tissues and contrast agent, in particular when contrast agent micro bubbles observation is key for diagnosis for example. These techniques take advantages of the non-linearity of the contrast agent response compared to the linear response of the tissue.

Amplitude Modulation (AM) is one of those techniques that consists in exciting the medium with successive pulses characterized by various intensities. For instance, the pulses as described above in context of figure 4 may be used. One pulse called "FULL" is generated by all the probe transducers of a given aperture and the two other pulses called "EVEN" and "ODD" are generated by half of the selected transducers (with one transducer out of two as an example).

Amplitude Modulation may also be performed by modifying other characteristics of the transmit beam such as the amplitude of the emitted pulse (by varying the voltage of the exciting transducer element(s)), the duty cycle (percent of the transmit period of the ultrasound wave during which transducer element(s) are excited by an electrical signal).

For each pulse, a beamforming process may be performed in order to estimate the reflectivity of the insonified region. Complex IQ maps associated to each pulse are then obtained called $I_{full}(r)$ (i.e. $I_1(r)$), $I_{EVEN}(r)$ (i.e. $I_2(r)$) and $I_{ODD}(r)$ (i.e. $I_3(r)$).

The CEUS complex image called $I_{ceus}(r)$ results from the coherent summation of the beamformed maps:

$$I_{ceus}(\mathbf{r}) = I_1(\mathbf{r}) - (I_2(\mathbf{r}) + I_3(\mathbf{r})) \qquad (1a)$$

The modulus of this image may then be log-converted, potentially filtered and stored, sent to another device, and/or displayed on a screen or using any display technic (such as hologram, connected glasses, ...).

By using half of the selected transducers, the intensity levels of the second and third transmitting pulses are approximatively half of the first pulse. On the one hand, the medium generally may have a linear response. The coherent summation of equation (1a) then deeply cancels their associated responses in the CEUS map. To the other hand, the non-linear response of the contrast agent does not fully cancel their response when performing same summation.

The non-linearity of the contrast agent induces a phase lag between the "FULL" and the "ODD" or "EVEN" pulses. Their associated backscattered signals are then no longer in phase, which implies that the cancellation doesn't fully operate, that is to say, signals does not compensate.

The present disclosure deals with taking benefits of this phase lag to enhance the quality of the CEUS images. This enhancement in particular results from the potential combination of two different processes, one improves the signals coming from the contrast agent, while the other one reduces the signals level generated by tissues and electronic noise.

Prior to each of the above-mentioned process, the complex maps $I_1(\mathbf{r})$, $I_2(\mathbf{r})$ and $I_3(\mathbf{r})$ may be first computed, and the contrast agent may be segmented.

Fig. 5 schematically shows the procedure of determining the first segmentation map $\phi_1^{seg}(\mathbf{r})$ according to the present disclosure. In order to segment the contrast agent, a first operation may consist in measuring the phase lag between the full and the sum of the ODD and EVEN IQ data. This can be as example achieved by measuring the phase of the scalar product between $I_1(\mathbf{r})$ and the sum of $I_2(\mathbf{r})$ and $I_3(\mathbf{r})$:

$$\phi_1(\mathbf{r}) = \arg[I_1(\mathbf{r}) \times \text{conj}(I_2(\mathbf{r}) + I_3(\mathbf{r}))] \quad (2)$$

Linear scatterers such as soft tissues should generate a phase lag $\phi_1$ that is close to $\pi$, meaning that $I_1$ and $(I_2 + I_3)$ are then more in phased. On the contrary, contrast agents may induce a phase lag of for example around -2 rad. Based on this observation, a segmentation may be done to isolate the contrast agent that are associated with a given phase lag:

$$\phi_1^{seg}(\mathbf{r}) = \begin{pmatrix} 1, & \text{if} & \phi_1^{min} < \phi_1(\mathbf{r}) < \phi_1^{max} \\ 0, & \text{else} \end{pmatrix} (3)$$

Exemplary values may comprise $\phi_1^{min} = -2.5$ rad and/or $\phi_1^{max} = -0.5$ rad. However, the values may also differ from on case to another and depending on medium, contrast agent(s), and other parameters. This segmentation may capture the contrast agent 20. As shown in fig 5, the contrast agent 20 is then shown as more obvious, i.e. has a better contrast in the first segmentation map $\phi_1^{seg}(\mathbf{r})$. In case an image is generated based on the map, it is thus advantageously more visible.

However, it is possible that on the edge (i.e. in peripheral areas of the insonified region of the medium) and at large depth, it also captures some electronic noise 21. Indeed, this noise is random and unpredictable. A part of it may thus fall within the selecting range $[\phi_1^{min}, \phi_1^{max}]$.

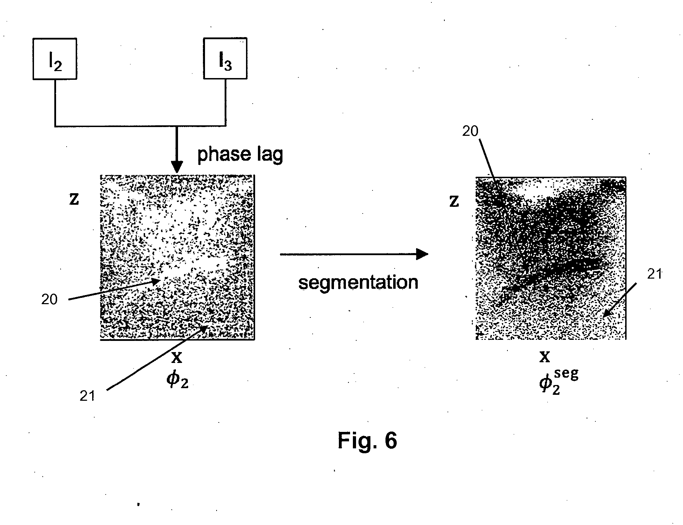

Fig. 6 schematically shows the procedure of determining the second segmentation map $\phi_2^{seg}(\mathbf{r})$ according to the present disclosure. To separate electronic noise 21 from contrast agent(s), the phase difference between $I_{ODD}(\mathbf{r})$ and $I_{EVEN}(\mathbf{r})$ may then be investigated. This may be done by measuring the scalar product between $I_2$ and $I_3$:

$$\phi_2(\mathbf{r}) = \arg[I_2(\mathbf{r}) \times \text{conj}(I_3(\mathbf{r}))] \quad (2)$$

$\phi_2$ may as a result be close to 0 for points associated for example to a central area of the insonified region of the medium and for the contrast agent (i.e. the information of interest). Indeed, the intensity level received by each point is the same for the ODD and EVEN pulse. As a result, only electronic noise may produce a phase lag between those two pulses, while contrast agent and tissue signals may both be in phase. A second segmentation can then be done:

$$\phi_2^{seg}(\mathbf{r}) = \begin{pmatrix} 1, \text{if} & |\phi_2(\mathbf{r})| > \phi_2^{max} \\ 0, \text{else} \end{pmatrix} \tag{3}$$

An exemplary value may comprise $\phi_2^{max} = 0.5$ rad. However, the value may also differ.

By combining $\phi_1^{seg}(\mathbf{r})$ and $\phi_2^{seg}(\mathbf{r})$, a third segmentation map $\phi_3^{seg}(\mathbf{r})$ may be formed that can better segment the contrast agent:

$$\phi_3^{seg}(\mathbf{r}) = \begin{pmatrix} 1, & \text{if} & \phi_1^{seg}(\mathbf{r}) = 1 \text{ and } \phi_2^{seg}(\mathbf{r}) = 0 \\ 0, & \text{else} \end{pmatrix} \tag{4}$$

Accordingly, the physical characteristic may be determined by:

$$I_{ceus}^+(\mathbf{r}) = (I_1(\mathbf{r}) - (I_2(\mathbf{r}) + I_3(\mathbf{r}))) \times \phi_3^{seg}(\mathbf{r}) \tag{1b}$$

In case there remains still some noise in the considered signal, it may be removed using as example a spatial filter as it is more scattered than the contrast agent signal.

In one option the contrast of the IQ data sets may be further enhanced. For example, as already noted above, the contrast agent phase lag $\phi_1$ may be known based on current medium, contrast agent(s) and/or data considered, e.g. may have a value of around -2 rad. Moreover, as already noted above, the CEUS image results from the coherent summation of $I_1(\mathbf{r})$ with $I_2(\mathbf{r}) + I_3(\mathbf{r})$. Consequently, a phase lag of -2 may not produce an optimal coherent summation. To enhance it, an additional phase lag may be introduced during the construction of $I_{ceus}(\mathbf{r})$ such as an optimal coherent summation occurred. This phase lag is as example applied based on the segmentation map $\phi_3$

$$I_{ceus}^{++}(\mathbf{r}) = I_1(\mathbf{r}) \times e^{-i\,\phi_1(\mathbf{r}) \times \phi_3^{seg}(\mathbf{r})} - (I_2(\mathbf{r}) + I_3(\mathbf{r})) \tag{1c}$$

Thanks to the contrast agent segmentation $\phi_3^{seg}(\mathbf{r})$, the contrast agent signal may be enhanced without modifying the tissue signals within the resulting IQ data. For example, the average contrast agent signals may be enhanced by approximatively 3 dB and up to 10 dB.

In a further option, noise and/or medium cancelation may be optimized. For signals that have been detected as noise (e.g. $\phi_2$), a phase lag such as $I_1$ may be inserted and as a consequence the sum of $I_2$ and $I_3$ is in opposite phase:

$$I_{ceus}^-(\mathbf{r}) = I_1(\mathbf{r}) \times e^{-i\,(\pi - \phi_1(\mathbf{r})) \times \phi_2^{seg}(\mathbf{r})} - (I_2(\mathbf{r}) + I_3(\mathbf{r})) \tag{1d}$$

and in a further enhancement. This process may be applied to any signal that is not considered as contrast agent $(1-\phi_3)$:

$$I_{ceus}^{--}(\mathbf{r}) = I_1(\mathbf{r}) \times e^{-i\,(\pi - \phi_1(\mathbf{r})) \times (1 - \phi_3^{seg}(\mathbf{r}))} - (I_2(\mathbf{r}) + I_3(\mathbf{r})) \tag{1e}$$

For example, the cancellation may reduce the noise level by approximatively 1.5 dB.

Finally, both the filtering and enhancing process may be combined to further improve the data quality of the determined physical characteristic, i.e. by:

$$I_{ceus}^{++-}(\mathbf{r}) = I_1(\mathbf{r}) \times e^{-i\,\phi_1(\mathbf{r}) \times \phi_3^{seg}(\mathbf{r})} \times e^{-i\,(\pi - \phi_1(\mathbf{r})) \times \phi_2^{seg}(\mathbf{r})} - \left(I_2(\mathbf{r}) + I_3(\mathbf{r})\right) \quad (1f)$$

and in a further enhancement by :

$$I_{ceus}^{++--}(\mathbf{r}) =$$
$$I_1(\mathbf{r}) \times e^{-i\,\phi_1(\mathbf{r}) \times \phi_3^{seg}(\mathbf{r})} \times e^{-i\,(\pi - \phi_1(\mathbf{r})) \times \left(1 - \phi_3^{seg}(\mathbf{r})\right)} - \left(I_2(\mathbf{r}) + I_3(\mathbf{r})\right) \quad (1g).$$

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Although the present disclosure herein has been described with reference to particular embodiments, it is to be understood that these embodiments are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

## Claims

**1.** A method for determining a physical characteristic of a medium (11), comprising:

processing (d) ultrasound signal data of the medium associated to at least three emitted ultrasound pulses for respectively providing at least three in-phase and quadrature phase (IQ) data sets $I_1(r)$, $I_2(r)$, $I_3(r)$, the at least three emitted ultrasound pulses comprising a first emitted pulse having a first intensity and at least two supplementary emitted pulses having each a second intensity, wherein a sum of the second intensities corresponds to the first intensity,
determining (e) the physical characteristic as a function of:

a first phase lag between the first IQ data set $I_1(r)$ and a sum of the at least two further IQ data sets $I_2(r)$, $I_3(r)$, and/or
a second phase lag between the at least two further IQ data sets $I_2(r)$, $I_3(r)$.

**2.** The method according to claims 1, wherein
determining (e) the physical characteristic further comprises:
setting the first IQ data set $I_1(r)$ in-phase with the sum of the at least two further IQ data sets $I_2(r)$, $I_3(r)$ by introducing a compensation phase lag to the first IQ data set $I_1(r)$ and/or to the sum of the at least two further IQ data sets $I_2(r)$, $I_3(r)$.

**3.** The method according to claim 1 or 2, wherein
determining (e) the physical characteristic further comprises:
setting the first IQ data set $I_1(r)$ in opposite phase to the sum of the at least two further IQ data sets $I_2(r)$, $I_3(r)$ by introducing pi minus the compensation phase lag.

**4.** The method according to any one of the preceding claims, wherein
the first phase lag $\phi_1(\mathbf{r})$ between the first IQ data set $I_1(r)$ and a sum of the at least two further IQ data sets $I_2(r)$, $I_3(r)$ is measured by:

a difference between the phase of $I_1(r)$ and the phase of the sum of $I_2(r)$, $I_3(r)$, or the phase of the scalar product between $I_1(r)$ and a sum of $I_2(r)$, $I_3(r)$, and/or by:

$$\phi_1(\mathbf{r}) = \arg[I_1(\mathbf{r}) \times \mathrm{conj}(I_2(\mathbf{r}) + I_3(\mathbf{r}))] \quad (2)$$

, and/or
the compensation phase lag is determined as a function of the first phase lag $\phi_1(r)$.

5. The method according to any one of the preceding claims, wherein the second phase lag $\phi_2(\mathbf{r})$ between the at least two further IQ data sets $I_2(r)$ and $I_3(r)$ is measured by: a difference between the phase of $I_2(r)$ and the phase of $I_3(r)$, or a phase of the scalar product between $I_2(r)$ and $I_3(r)$, and/or by:

$$\phi_2(\mathbf{r}) = \arg\big[I_2(\boldsymbol{r}) \times \mathrm{conj}\big(I_3(\boldsymbol{r})\big)\big] \quad (4).$$

6. The method according to any one of the preceding claims, wherein determining (e) the physical characteristic further comprises:

   determining a first segmentation map as a function of the first phase lag and/or a second segmentation map as a function of the second phase lag, wherein
   the physical characteristic is determined as a function of the first and/or the second segmentation map.

7. The method according to the preceding claim, wherein
   the physical characteristic is determined as a function of a third segmentation map generated by combining the first segmentation map and the second segmentation map, and/or by removing the content of the second segmentation map from the first segmentation map.

8. The method according to any one of the preceding claims 6 and 7,
   wherein

   the first segmentation map is configured to segment an area of interest of the medium, and/or
   the second segmentation map is configured to detect and/or reduce noise in the IQ data sets $I_1(r)$, $I_2(r)$, $I_3(r)$.

9. The method according to any one of the preceding claims 6 to 8,
   wherein

   the first segmentation map is determined by:

$$\phi_1^{\mathrm{seg}}(\mathbf{r}) = \begin{pmatrix} 1, & \text{if} & \phi_1^{\min} < \phi_1(\mathbf{r}) < \phi_1^{\max} \\ 0, & \text{else} \end{pmatrix} \quad (3),$$

   wherein $\phi_1^{\min}$ and $\phi_1^{\max}$ are predefined values, and/or
   the second segmentation map is determined by:

$$\phi_2^{seg}(\boldsymbol{r}) = \begin{pmatrix} 1, if & |\phi_2(\boldsymbol{r})| > \phi_2^{max} \\ 0, else \end{pmatrix} (5),$$

   wherein $\phi_2^{\max}$ is a predefined value, and/or
   the third segmentation map is determined by:

$$\phi_3^{\text{seg}}(\mathbf{r}) = \begin{pmatrix} 1, & \text{if} & \phi_1^{\text{seg}}(\mathbf{r}) = 1 \text{ and } \phi_2^{\text{seg}}(\mathbf{r}) = 0 \\ 0, & \text{else} \end{pmatrix} \qquad (6).$$

10. The method according to any one of the preceding claims, wherein the physical characteristic is determined based on at least one of:

a predefined contrast-enhanced ultrasound method,
a coherent summation of the IQ data sets $I_1(r)$, $I_2(r)$, $I_3(r)$,
the equation (1b):

$$I_{\text{ceus}}^{+}(\mathbf{r}) = \left( I_1(\mathbf{r}) - \left( I_2(\mathbf{r}) + I_3(\mathbf{r}) \right) \right) \times \phi_3^{\text{seg}}(\mathbf{r}),$$

the equation (1c):

$$I_{\text{ceus}}^{++}(\mathbf{r}) = I_1(\mathbf{r}) \times e^{-i\,\phi_1(\mathbf{r}) \times \phi_3^{\text{seg}}(\mathbf{r})} - \left( I_2(\mathbf{r}) + I_3(\mathbf{r}) \right),$$

the equation (1d):

$$I_{\text{ceus}}^{-}(\mathbf{r}) = I_1(\mathbf{r}) \times e^{-i\,(\pi-\,\phi_1(\mathbf{r})) \times \phi_2^{\text{seg}}(\mathbf{r})} - \left( I_2(\mathbf{r}) + I_3(\mathbf{r}) \right),$$

the equation (1e):

$$I_{\text{ceus}}^{--}(\mathbf{r}) = I_1(\mathbf{r}) \times e^{-i\,(\pi-\,\phi_1(\mathbf{r})) \times \left(1-\phi_3^{\text{seg}}(\mathbf{r})\right)} - \left( I_2(\mathbf{r}) + I_3(\mathbf{r}) \right),$$

the equation (1f):

$$I_{\text{ceus}}^{++-}(\mathbf{r}) = I_1(\mathbf{r}) \times e^{-i\,\phi_1(\mathbf{r}) \times \phi_3^{\text{seg}}(\mathbf{r})} \times e^{-i\,(\pi-\,\phi_1(\mathbf{r})) \times \phi_2^{\text{seg}}(\mathbf{r})} - \left( I_2(\mathbf{r}) + I_3(\mathbf{r}) \right),$$

and the equation (1g):

$$I_{\text{ceus}}^{++--}(\mathbf{r}) =$$
$$I_1(\mathbf{r}) \times e^{-i\,\phi_1(\mathbf{r}) \times \phi_3^{\text{seg}}(\mathbf{r})} \times e^{-i\,(\pi-\,\phi_1(\mathbf{r})) \times \left(1-\phi_3^{\text{seg}}(\mathbf{r})\right)} - \left( I_2(\mathbf{r}) + I_3(\mathbf{r}) \right).$$

11. The method according to any one of the preceding claims, further comprising before processing (d) ultrasound signal data:

transmitting (b) an emitted sequence (ES) of ultrasound waves (We) into the medium (11), the emitted sequence (ES) comprising the first emitted pulse and the at least two supplementary emitted pulses, and
receiving (c) a response sequence (RS) of ultrasound waves (Wr) from the medium, wherein the ultrasound signal data are based on the response sequence (RS) of ultrasound waves (Wr), and/or
the method comprising before sending (b) the emitted sequence (ES) of ultrasound waves (We):

introducing (a) a contrast agent into the medium,
wherein the first segmentation map is configured to segment the contrast agent in the medium.

12. The method according to any one of the preceding claims, wherein

the at least two supplementary emitted pulses are spatially offset from each other with respect to the medium, and/or

the second and third pulse are emitted with different aperture modulations, and/or

a set of transducer elements used for emitting the first pulse is split into at least two sub-sets which are respectively used to emit the at least two supplementary pulses, and/or

a first sub-set corresponds to the odd numbers of the transducer elements used for emitting the first supplementary pulse, and a second sub-set corresponds to the even numbers of the transducer elements used for emitting the second supplementary pulse.

13. A method for determining physical characteristics of a plurality of regions of a medium (11), comprising:
repeatedly performing the method according to any one of the preceding claims, wherein in each iteration ultrasound signal data of another region is processed.

14. The method according to the preceding claim, wherein

at least one of the first, second and third segmentation map are determined as a function of the plurality of regions, and/or

an image is formed based on the determined physical characteristics of the plurality of regions.

15. A computer program comprising computer-readable instructions which when executed by a data processing system cause the data processing system to carry out the method according to any one of preceding method claims.

16. A system for determining a physical characteristic of a medium (11), comprising a processing unit configured to:

process ultrasound signal data of the medium associated to at least three emitted ultrasound pulses for respectively providing at least three in-phase and quadrature phase (IQ) data sets $I_1(r)$, $I_2(r)$, $I_3(r)$, the at least three emitted ultrasound pulses comprising a first emitted pulse having a first intensity and at least two supplementary emitted pulses having each a second intensity, wherein a sum of the second intensities corresponds to the first intensity,

determine the physical characteristic as a function of:

a first phase lag between the first IQ data set $I_1(r)$ and a sum of the at least two further IQ data sets $I_2(r)$, $I_3(r)$, and/or

a function of a second phase lag between the at least two further IQ data sets $I_2(r)$, $I_3(r)$.

**Fig. 1**

Fig. 2

Fig. 3

# Loop over pulses

12

20

Pulse 1 : FULL     Pulse 2 : EVEN     Pulse 3 : ODD

P1       P2       P3

Scanning line

P1'       P2'       P3'

Loop over scanning line

## Fig. 4

$I_1$        $I_2 + I_3$

phase lag

20

Z       Z

20       segmentation       21

X         X

$\phi_1$        $\phi_1^{seg}$

## Fig. 5

**Fig. 6**

Europäisches
Patentamt

European
Patent Office

Office européen
des brevets

**EUROPEAN SEARCH REPORT**

Application Number

EP 21 31 5228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/069504 A1 (WILKENING WILKO G [DE] ET AL) 10 April 2003 (2003-04-10) * paragraphs [0014], [0018] – [0022], [0026], [0029] – [0031], [0068] – [0071]; figure 1 * | 1–16 | INV. A61B8/08 G01S7/52 G01S15/10 G01S15/89 |
| X | US 2003/204142 A1 (BROCK-FISHER GEORGE A [US] ET AL) 30 October 2003 (2003-10-30) * paragraphs [0049], [0073] – [0078]; figures 1,2,7,9 * | 1–16 | |
| A | US 2020/397412 A1 (NOGUCHI MASAFUMI [JP]) 24 December 2020 (2020-12-24) * paragraphs [0066] – [0068]; figures 1,5 * | 1–16 | |

| | | | TECHNICAL FIELDS SEARCHED (IPC) |
|---|---|---|---|
| | | | A61B G01S |

The present search report has been drawn up for all claims

| Place of search | Date of completion of the search | Examiner |
|---|---|---|
| Munich | 31 March 2022 | Theißing, Nikolaus |

EPO FORM 1503 03.82 (P04C01)

## ANNEX TO THE EUROPEAN SEARCH REPORT
## ON EUROPEAN PATENT APPLICATION NO.

EP 21 31 5228

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2022

| Patent document cited in search report | | Publication date | Patent family member(s) | | Publication date |
|---|---|---|---|---|---|
| US 2003069504 | A1 | 10-04-2003 | DE | 10246353 A1 | 24-04-2003 |
| | | | US | 2003069504 A1 | 10-04-2003 |
| US 2003204142 | A1 | 30-10-2003 | AU | 2003214575 A1 | 10-11-2003 |
| | | | EP | 1501419 A1 | 02-02-2005 |
| | | | JP | 2005523743 A | 11-08-2005 |
| | | | US | 2003204142 A1 | 30-10-2003 |
| | | | US | 2004030253 A1 | 12-02-2004 |
| | | | WO | 03090624 A1 | 06-11-2003 |
| US 2020397412 | A1 | 24-12-2020 | EP | 3777699 A1 | 17-02-2021 |
| | | | JP | 6963677 B2 | 10-11-2021 |
| | | | JP | WO2019189386 A1 | 18-02-2021 |
| | | | US | 2020397412 A1 | 24-12-2020 |
| | | | WO | 2019189386 A1 | 03-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3097432 A1

**Non-patent literature cited in the description**

- **TREMBLAY-DARVEAU C ; SHEERAN PS ; VU CK et al.** The Role of Microbubble Echo Phase Lag in Multipulse Contrast-Enhanced Ultrasound Imaging. *IEEE Trans Ultrason Ferroelectr Freq Control,* 2018, vol. 65 (8), 1389-1401